# EUROPÄISCHE PATENTANMELDUNG

(11) **EP 1 889 584 A1**
(43) Veröffentlichungstag der Anmeldung: **20.02.2008**
(21) Anmeldenummer: 06017161.8
(22) Anmeldetag: 17.08.2006
(51) Int. Cl.: A61B 19/00

(54) **Bestimmung des Drehzentrums eines Implantats**

(71) Anmelder: BrainLAB AG, 85622 Feldkirchen (DE)
(72) Erfinder: Drumm, Peter, 81543 München (DE)
(74) Vertreter: Engelhard, Maximilian

(57) **Zusammenfassung**

Implantatdrehzentrum-Bestimmungsgerät zum Bestimmen des Drehzentrums eines medizinischen Implantats, mit einem eine sphärische Oberfläche aufweisenden Anlageglied (20,30) zum Anlegen eines sphärischen Implantats, mit Markern (12,14,16), die ortsfest relativ zum Anlageglied angeordnet sind sowie ein Verfahren zum Bestimmen eines Implantatdrehzentrums mittels des Implantatdrehzentrum-Bestimmungsgeräts bei dem ein Implantatdrehzentrum-Bestimmungsgerät ausgewählt wird, dessen sphärische Oberfläche einen Radius hat, die mit dem Radius der sphärischen Oberfläche des Implantats übereinstimmt, die sphärische Oberfläche des Implantatdrehzentrum-Bestimmungsgeräts an die komplementär ausgebildete sphärische Oberfläche des Implantats angelegt wird, mit der Detektionseinrichtung mindestens eine Lage der Marker detektiert wird und mittels der Datenverarbeitungseinrichtung basierend auf einer Vielzahl von detektierten Lagen der Marker oder basierend auf einer detektierten Lage der Marker und basierend auf der bekannten relativen Lage zwischen sphärischem Mittelpunkt des Anlageglieds und den Markern der sphärische Mittelpunkt des Implantats bestimmt wird.

## Beschreibung

Die vorliegende Erfindung betrifft ein Implantatdrehzentrum-Bestimmungsgerät, das die Bestimmung des Drehzentrums eines Implantats erlaubt, ein Implantatdrehzentrum-Bestimmungssystem, das das Implantatdrehzentrum-Bestimmungsgerät verwendet und dessen Marker detektiert sowie die detektierten Signale verarbeitet, um das Drehzentrum zu bestimmen sowie ein entsprechendes Verfahren zur Bestimmung des Implantatdrehzentrums mit Hilfe des Implantatdrehzentrum-Bestimmungssystems.

Aufgabe der Erfindung ist es, die Bestimmung des Drehzentrums von sphärischen Implantaten, deren Geometrie insbesondere unbekannt ist, zu ermöglichen.

Vorstehende Aufgabe wird durch die Gegenstände der unabhängigen Ansprüche gelöst. Vorteilhafte Weiterbildungen gehen aus den abhängigen Ansprüchen hervor.

Vorteilhaft erlaubt die vorliegende Erfindung weiter, die Berechnung einer Beinlängendifferenz bei einer Hüftendoprothese, die Bestimmung einer Vorwärtsneigung des Femurs, die Bestimmung einer medial-lateral Verschiebung und/oder einer cranial-craudalen Verschiebung nach der Implantation.

Der Begriff "sphärische Oberfläche" bezeichnet hierin eine Oberfläche, die konvex oder konkav sphärisch ausgebildet ist. Die Oberfläche stimmt also zum Teil mit einer Kugel überein. Der Mittelpunkt dieser Kugel ist der sphärische Mittelpunkt der sphärischen Oberfläche. Der sphärische Mittelpunkt der sphärischen Oberfläche des Implantats stimmt mit dem Drehzentrum des Implantats überein.

Die hierin beschriebenen Marker können direkt an dem Anlageglied angebracht sein oder können eine vom Anlageglied separate Markereinrichtung bilden. Eine Markereinrichtung umfasst also die Marker und weitere Elemente wie z.B. Arme, die die Marker in einer fest vorgegebenen Lage miteinander verbinden.

Das erfindungsgemäße Implantatdrehzentrum-Bestimmungsgerät wird weiter unten noch näher beschrieben. Es erlaubt in Verbindung mit einer Detektionseinrichtung, die die Marker detektiert und einer Datenverarbeitungseinrichtung, die die Detektionssignale verarbeitet, die Bestimmung des Drehzentrums.

Erfindungsgemäß werden also mittels einer Detektionseinrichtung (z. B. Kamera oder Ultraschalldetektor) Markereinrichtungen detektiert. Die Markereinrichtungen umfassen typischerweise drei Marker, die in fester und vorbestimmter relativer Lage zueinander angeordnet sind und insbesondere mechanisch verbunden sind. Die Marker können passive oder aktive Marker sein, wobei die passiven Marker Signale (z.B. Wellen und/oder Strahlung) reflektieren, die in ihre Richtung ausgesendet werden und die aktiven Marker selbst Ursprung der Signale (z.B. Strahlung und/oder Wellen) sind. Die von den (aktiven oder passiven) Markern ausgehenden Signale, bei denen es sich z.B. um Wellensignale oder Strahlungssignale handeln kann, werden von einer Detektionsvorrichtung (z. B. Kamera) detektiert. Um eine Position der Markereinrichtung relativ zu der Detektionseinrichtung festzulegen, wird dabei vorzugsweise die Markereinrichtung bewegt, um der Detektionseinrichtung verschiedene Ansichten der Markereinrichtung zu bieten. Auf dieser Grundlage kann dann in bekannter Weise die relative Lage der Markereinrichtung relativ zu der Detektionseinrichtung, insbesondere in einem im Raum ruhenden Bezugssystem bestimmt werden. In diesem Zusammenhang wird auf die DE 196 39 615 A1 und die entsprechende US-Veröffentlichung 6,351,659 hingewiesen, die hiermit durch Bezugnahme in die vorliegende Offenbarung aufgenommen wird.

Die Lage der Markereinrichtung wird bevorzugt durch die Position der Markereinrichtung in einem vorbestimmen Bezugssystem bestimmt. Vorzugsweise wird als Bezugssystem ein Bezugssystem verwendet, in dem die Detektionseinrichtung ruht. Insbesondere wird die Lage der Markereinrichtung durch die Positionen der Marker, insbesondere der Mittelpunkte der Marker in einem Bezugssystem bestimmt. Die Positionen können zum Beispiel mit kartesischen Koordinaten oder Kugelkoordinaten beschrieben werden. Die relative Lage von einem Teil (z. B. Detektionseinrichtung oder Markereinrichtung) zu einem anderen Teil (z. B. Markereinrichtung) kann insbesondere durch Raumwinkel und/oder Abstände und/oder Koordinaten (in einem Bezugssystem) und/oder Vektoren beschrieben werden und wird vorzugsweise aus dem die Lage beschreibenden Positionen z. B. mittels eines Programms errechnet, das auf einem Computer läuft.

Der hierin verwendete Begriff "relative Lage" oder der Ausdruck "Lage eines Teils A relativ zu einem Teil B" umfasst also den Begriff der relativen Positionen zwischen den zwei Teilen, insbesondere zwischen den Markereinrichtungen und/oder deren Markern oder zwischen einer Markereinrichtung (oder deren Markern) und der Detektionseinrichtung. Insbesondere werden Schwerpunkte oder Mittelpunkte der Teile als punktförmiger Bezugspunkt zum Festlegen einer Position gewählt. Ist die Position eines Teils in einem Bezugssystem bekannt, so kann basierend auf der relativen Lage zweier Teile aus der Position eines der zwei Teile die Position des anderen der zwei Teile berechnet werden.

Insbesondere falls die Markereinrichtung nur zwei Marker umfasst, ist bevorzugt eine Startposition bekannt und die Markersystem erlaubt dann die Verfolgung der Lage der Markereinrichtung bei Bewegung der Markereinrichtung im Raum.

Die Markereinrichtung gemäß der vorliegenden Erfindung umfasst also vorzugsweise mindestens zwei Marker, insbesondere und bevorzugt drei Marker und kann natürlich auch mehr als drei Marker umfassen. Die Abmessungen der Marker und die relativen Lagen der Marker zueinander sind vorzugsweise und liegen insbesondere als vorbekannte Daten einer Datenverarbeitungseinrichtung vor. Vorzugsweise ist auch die Form der Marker bekannt.

Weiter umfasst das erfindungsgemäße Markernavigationssystem vorzugsweise eine Detektionseinrichtung, die Signale von den mindestens zwei Markern (z. B. von drei Markern) detektiert. Wie zuvor ausgeführt handelt es sich hierbei um von den Markern ausgehende Signale, die entweder aktiv von den Markern ausgesendet werden oder von den Markern reflektiert werden. Im letzteren Fall ist vorzugsweise weiter eine Signalsendequelle beispielsweise eine Infrarotlichtquelle vorgesehen, die Signale (z. B. Ultraschallwellen oder Infrarotlicht) in Richtung auf die passiven Marker (kontinuierlich oder gepulst) aussendet, wobei die passiven Marker die Signale reflektieren. Eine Datenverarbeitungseinrichtung, insbesondere ein Computer erlaubt die Berechnung der relativen Lage der Markereinrichtung relativ zur Detektionseinrichtung, insbesondere die Berechnung der Lage der Markereinrichtung in einem Bezugssystem, indem die Detektionseinrichtung ruht, also beispielsweise in einem Bezugssystem, das in einem Operationssaal ruht.

Die erfindungsgemäße Datenverarbeitungseinrichtung ist vorzugsweise ausgebildet, um Berechnungs- und/oder Bestimmungsoperationen durchzuführen. Erfindungsgemäß werden durch die Datenverarbeitungseinrichtung die Lagen der Markereinrichtungen basierend auf den detektierten Signalen, die von den Markereinrichtungen ausgehen, berechnet. Vorzugsweise sind die Gegenstände (Körperstruktur oder Instrument), an denen die Markereinrichtungen angebracht sind, kalibriert. Dies bedeutet, dass die relativen Lagen zumindest zwischen Teilen des Gegenstandes und der Markereinrichtung, die an dem Gegenstand angebracht ist, bekannt sind und/oder insbesondere in der Datenverarbeitungseinrichtung abgespeichert sind bzw. eingegeben werden, so dass insbesondere Anzeigesignale, die die Lagen der Gegenstände beschreiben, basierend auf den Lagen der Markereinrichtungen bestimmt werden können. Die Lagen der Markereinrichtungen werden bevorzugt relativ zu der Detektionseinrichtung berechnet, also in einem Bezugssystem, in dem die Detektionseinrichtung ruht. Natürlich können die Lagen auch in einem anderen Bezugssystem berechnet werden, z.B. in einem Bezugssystem, in dem der Patient ruht und/oder in dem eine der Markereinrichtungen ruht.

Das erfindungsgemäße Implantatdrehzentrum-Bestimmungsgerät umfasst, wie oben ausgeführt, ein Anlageglied, das an ein Implantat anzulegen ist. Dieses Implantat ist sphärisch, d.h., ein Teil der Oberfläche des Implantats entspricht der Oberfläche einer Kugel. Wie oben ausgeführt, ist die Aufgabe der Erfindung, den sphärischen Mittelpunkt des Implantats zu bestimmen. Hierzu wird eine sphärisch gestaltete Oberfläche des Anlageglieds an die sphärische Oberfläche des Implantats angelegt. Dabei kann gemäß einem ersten Fall die sphärischen Oberfläche des Implantats konvex sein und die sphärische Oberfläche des Anlageglieds entsprechend dazu (also komplementär dazu) konkav ausgebildet sein. Gemäß einem zweiten Fall ist dies umgekehrt. Die sphärische Oberfläche des Implantats ist konkav und die sphärische Oberfläche des Anlageglieds ist hierzu wiederum komplementär ausgebildet, also konvex. Dabei ist das Anlageglied so zu gestalten und insbesondere vom Anwender auszuwählen, dass die sphärische Oberfläche des Anlageglieds (möglichst) satt, d.h. berührend an der sphärischen Oberfläche des Implantats anliegt.

Die Marker des Implantatdrehzentrum-Bestimmungsgeräts sind bei der Anwendung des Implantatdrehzentrum-Bestimmungsgeräts ortsfest relativ zum Anlageglied. Die relative Lage relativ zu dem Anlageglied ist vorzugsweise bekannt. Besonders bevorzugt ist die relative Lage der Marker relativ zum sphärischen Mittelpunkt der sphärischen Oberfläche des Anlageglieds bekannt. Bestimmt man also die Lage der Marker, lässt sich hieraus die Lage des sphärischen Mittelpunkts des Anlageglieds berechnen. Da das Anlageglied mit seiner sphärischen Oberfläche satt an der sphärischen Oberfläche des Implantats anliegt, stimmen die beiden sphärischen Mittelpunkte miteinander überein. Ist also die Lage der Marker in einem Bezugssystem bekannt (z.B. kann der Operationssaal das Bezugssystem sein, in dem die Kamera ruht), so kann aus der Lage der Marker und aus der relativen Lage der Marker relativ zum sphärischen Mittelpunkt des Anlageglieds auch der sphärische Mittelpunkt des Implantats und somit die Lage des Drehzentrums bestimmt werden.

Bei einer Ausführungsform bilden die Marker eine Markereinrichtung bzw. sind Bestandteil einer Markereinrichtung. Die Markereinrichtung ist dabei vorzugsweise lösbar an dem Anlageglied angebracht, um verschieden gestaltete Markereinrichtungen (z.B. Referenzsterne) an dem Anlageglied anbringen zu können und um eine Reinigung des Anlageglieds zu erleichtern.

Wie bereits oben ausgeführt, wird das Anlageglied vorzugsweise satt an das Implantat angelegt. Um diese zu gewährleisten, sollte die Krümmung der sphärischen Oberfläche des Anlageglieds mit der Krümmung der sphärischen Oberfläche des Implantats übereinstimmen und/oder zumindest zu 90 % oder mehr übereinstimmen. Hierzu können verschiedene Implantatdrehzentrum-Bestimmungsgeräte vorgesehen werden, die einem Bediener (z.B. Operateur) zur Verfügung stehen und die Anlageglieder mit sphärischen Oberflächen unterschiedlicher Krümmung umfassen. Besonders bevorzugt wird das Implantatdrehzentrum-Bestimmungsgerät so gestaltet, dass das Anlageglied zweiteilig gestaltet ist, wobei ein Teil, das Anlageflächenglied, austauschbar gestaltet ist. Das Anlageglied umfasst also einen Träger und ein Anlageflächenglied, wobei verschiedenen Anlageflächenglieder mit dem Träger verbindbar sind, insbesondere lösbar verbindbar sind. Die Anlageflächenglieder können also vorzugsweise ausgetauscht werden, wobei verschiedene Anlageglieder sphärische Oberflächen unterschiedlicher Krümmung aufweisen. Der Träger ist vorzugsweise so gestaltet, dass daran die Marker angebracht sind oder eine Markereinrichtung angebracht ist, insbesondere lösbar angebracht ist.

Vorzugsweise ist das Anlageglied so gestaltet, dass Anlageflächenglieder mit wahlweise konvexer oder konkaver sphärischer Oberfläche und/oder verschiedener Ausgestaltung (z.B. anderer verschiedener Krümmungsradius der sphärischen Anlagefläche) daran angebracht werden können. Ein konkaves Anlageflächenglied ist dabei vorzugsweise schalenförmig ausgebildet und umfasst eine sphärische Vertiefung. Ein Anlageflächenglied mit konvexer sphärischer Oberfläche umfasst vorzugsweise an Anbringglied, das an dem Träger vorzugsweise lösbar angebracht wird. Vorzugsweise ist weiter vorgesehen ein Abstandsglied (z.B. Fortsatz), beispielsweise eine Stange oder Stutzen, die ein Kontaktflächenglied, z.B. ein Kopf mit sphärischer Oberfläche mit dem Anbringglied verbindet und umfasst und somit einen Abstand zwischen Träger und Kontaktflächenglied (Kopf) bewirkt. Das Kontaktflächenglied wird vom Bedienen mit dem konkaven, sphärischen Implantat in Kontakt gebracht.

Das erfindungsgemäße Implantatdrehzentrum-Bestimmungssystem umfasst das zuvor beschriebene Implantatdrehzentrum-Bestimmungsgerät und weiter eine Detektionseinrichtung und eine Datenverarbeitungseinrichtung. Die Detektionseinrichtung detektiert Signale von den Markern. Die detektierten Signale werden von der Datenverarbeitungseinrichtung verarbeitet, die aus den detektierten Signalen die Lage des sphärischen Mittelpunkts des Anlageglieds und damit das Drehzentrum des Implantats berechnet.

Die Datenverarbeitungseinrichtung kann die Lage des sphärischen Mittelpunkts auf verschiedene Arten bestimmen. Gemäß einem ersten Fall ist die relative Lage des sphärischen Mittelpunkts relativ zu den Markern (und zu der Markereinrichtung) bekannt. Hierbei bedeutet "bekannt", dass die der relativen Lage entsprechenden Daten in der Datenverarbeitungseinrichtung gespeichert sind und/oder in diese eingegeben werden. Diese Daten verarbeitet dann die Datenverarbeitungseinrichtung, um zusammen mit der detektierten Lage der Marker die detektierte Lage des sphärischen Mittelpunkts des Anlageglieds und damit des Implantats zu bestimmen. Hierzu wird das Implantatdrehzentrum-Bestimmungsgerät satt an die sphärische Oberfläche des Implantats angelegt, während die Signale von den Markern detektiert werden und dann zur Lagebestimmung des. Implantatdrehzentrum-Bestimmungsgeräts weiter verarbeitet werden.

Es kann jedoch auch die relative Lage zwischen dem sphärischen Mittelpunkt des Anlageglieds und den Markern unbekannt sein. In diesem Falle sollte das Implantatdrehzentrum-Bestimmungsgerät bewegt werden, während es in sattem Kontakt mit der sphärischen Oberfläche des Implantats ist. Die Trajektorien der Bewegung sollten eine sphärische Fläche aufspannen, also sollte z.B. eine Schwenkbewegung um zwei verschiedene Achsen vorzugsweise erfolgen. Vorzugsweise werden dabei zumindest drei verschiedene Lagen der Marker bzw. der Markereinrichtung detektiert. Durch numerische Verfahren wird z.B. bestimmt, ob diese verschiedene Lagen auf einer Kugeloberfläche liegen und falls ja, wird der Mittelpunkt der dieser Kugeloberfläche entsprechenden Kugel sowie vorzugsweise auch der Radius der Kugel bestimmt. Durch Bestimmung der Lage des Mittelpunktes ist somit der sphärische Mittelpunkt des Anlageglieds und auch der sphärischen Mittelpunkt der sphärischen Oberfläche des Implantats bestimmt.

Im Folgenden werden weitere Vorteile und Merkmale der Erfindung beschrieben. Verschiedene Merkmale unterschiedlicher Ausführungsformen können miteinander kombiniert werden. Bei der folgenden Beschreibung bezeichnen gleiche Bezugszeichen gleiche Teile soweit nicht anders bezeichnet.
- Fig. 1: zeigt ein Implantatdrehzentrum-Bestimmungsgerät zur Bestimmung des sphärischen Mittelpunkts von Implantaten mit konvexer sphärischer Oberfläche;
- Fig. 2: zeigt eine schematische Schnittansicht der Ausführungsform gemäß Fig. 1;
- Fig.3: zeigt eine Anwendung des erfindungsgemäßen Implantatdrehzentrum-Bestimmungssystems;
- Fig. 4: zeigt eine weitere Ausführungsform eines erfindungsgemäßen Implantatdrehzentrum-Bestimmungsgeräts, das die Bestimmung des sphärischen Mittelpunkts von Implantaten mit konkaver sphärischer Oberfläche erlaubt;
- Fig. 5: zeigt ein erfindungsgemäßes Implantatdrehzentrum-Bestimmungssystem, das das Implantatdrehzentrum-Bestimmungsgerät gemäß der Fig. 4 verwendet.

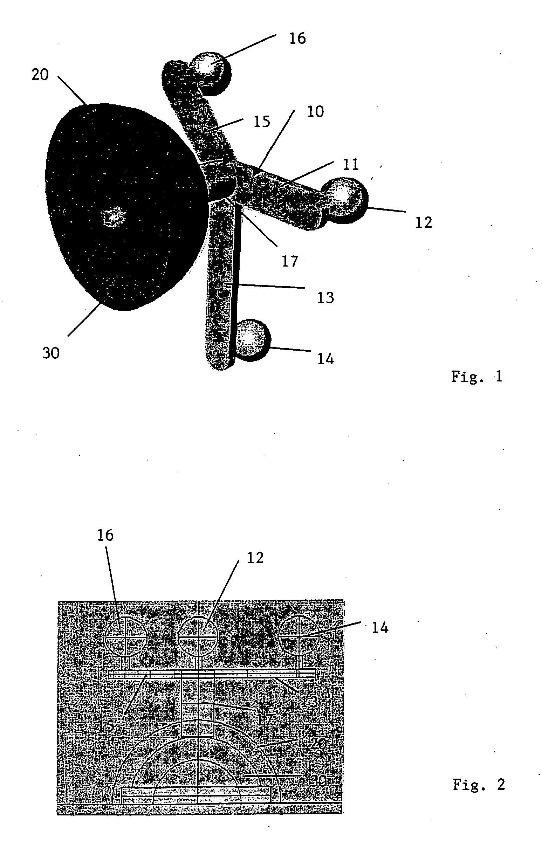

Fig. 1 zeigt ein Implantatdrehzentrum-Bestimmungsgerät gemäß der vorliegenden Erfindung (im vorliegenden kurz "ICORD" für "Implant Center of Rotation Determination Device"). Das ICORD umfasst Marker 12, 14 und 16, die ortsfest mit einem Träger 20 über Arme 11, 13 und 15 sowie einen Stutzten 17, der die Arme 11, 13 und 15 mit dem Träger 20 verbindet, verbunden sind. Die Arme 11, 13 und 15 sorgen dafür, dass die Marker 12, 14 und 16 relativ zueinander in einer bestimmten charakteristischen räumlichen Anordnung vorhanden sind. Die Marker 12, 14, 16 bilden zusammen mit den Armen 11, 13 und 15 sowie den Stutzen 17 eine Markereinrichtung. Alternativ können die Marker natürlich auch direkt an dem Träger 20 oder, falls kein Träger vorgesehen ist, direkt an dem Anlageflächenglied 30 angebracht sein, ohne dass Arme 11, 13 und 15 sowie ein Stutzen 17 vorgesehen sind. Vorteilhaft ist die Markereinrichtung 10 von dem Träger 20 lösbar und wieder damit ortsfest verbindbar. Somit können je nach Anwendung verschiedene Markereinrichtungen verwendet werden. Der Träger 20 ist nicht obligatorisch. Innerhalb des Trägers 20 ist ein Anlageflächenglied 30 vorgesehen. Ist kein Träger 20 vorhanden, können die Marker natürlich auch direkt an dem Anlageflächenglied 30 angebracht werden. Das Anlageflächenglied 30 bildet eine Fläche zum Anlegen eines sphärischen Körpers, insbesondere eines sphärischen Implantats.

Fig. 2 zeigt eine schematische Seiten- und Schnittansicht eines ICORD. Gleiche Teile sind wiederum mit gleichen Bezugszeichen bezeichnet. Erkennbar ist hier im Schnitt die sphärische Vertiefung, die durch das Anlageflächenglied 30 gebildet wird. Das Anlageflächenglied ist also konkav ausgebildet.

Wie aus der Schnittansicht in Fig. 2 ersichtlich ist, ist der Träger 20 als sphärische Schale ausgebildet und weist ebenfalls eine konkave sphärische Innenfläche auf, an der die Außenfläche des Anlageflächenglieds 30 satt anliegt. Das Anlageflächenglied 30 kann mit dem Träger 20 durch Presspassung verbunden sein. Alternativ oder zusätzlich kann beispielsweise eine Steckverbindung vorgesehen sein, bei der Fortsätze und Vertiefungen in dem Anlageflächenglied und dem Träger 20 ineinander greifen, um eine ortsfeste Verbindung zwischen dem Anlageflächenglied und dem Träger 20 zu gewährleisten. Die Verbindung ist vorzugsweise lösbar ausgebildet und kann z.B. eine Rastverbindung sein.

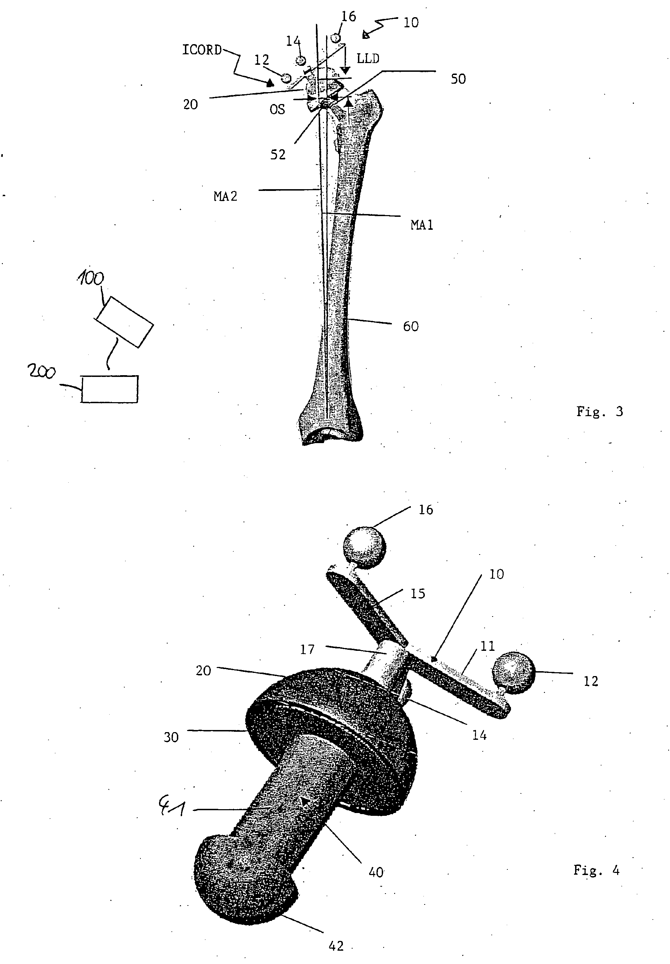

Fig. 3 zeigt einen Anwendungsfall für das ICORD. Der Kopf eines Oberschenkelknochens 60 ist reseziert. Ein Implantat 50 ist in den Oberschenkelknochen eingeführt, wie dies bei der sog. Schenkelhalsendoprothese durchgeführt wird. Das ICORD ist über einen sphärischen Kopf 52 des Implantats 50 gestülpt, so dass der konvexe sphärische Kopf 52 des Implantats 50 satt an der konkaven, sphärischen Innenfläche des ICORDs anliegt. Von den Marker 12, 14 und 16 werden Signale reflektiert, die von der Kamera 100 detektiert werden. Bei den Signale handelt es sich beispielsweise um Infrarotlicht, das von einem Sender kontinuierlich oder pulsweise ausgestrahlt wird. Der Sender kann beispielsweise auch in der Kamera 100 untergebracht sein. Die detektierten Signale werden von der Datenverarbeitungseinrichtung 200 weiter verarbeitet. Bei dem ICORD handelt es sich beispielsweise um eines, bei dem die relative Lage des sphärischen Mittelpunkts des Anlageflächenglieds relativ zu der Markereinrichtung 10 und also somit relativ zu den Markern 12, 14 und 16 bekannt ist. Da das ICORD satt an dem Implantatkopf 52 anliegt, kann somit aus der Detektion der Lage der Markereinrichtung durch die Kamera 100 mittels der Datenverarbeitungseinrichtung 200 die Lage des sphärischen Mittelpunkts bestimmt werden. Der sphärische Mittelpunkt des Anlageflächenglieds stimmt mit dem sphärischen Mittelpunkt des Implantatkopfes 52 überein, da der Implantatkopf 52 satt an dem Anlageflächenglied anliegt. Somit ist der sphärische Mittelpunkt des Implantatkopfs 52 durch das erfindungsgemäße Implantatdrehzentrum-Bestimmungssystem (im Folgenden kurze ICORS) bestimmbar, wobei dieses zusätzlich zum ICORD die Detektionseinrichtung 100 und die Datenverarbeitungseinrichtung 200 umfasst.

Vor der Operation wurde das Drehzentrum dadurch bestimmt, dass der Drehpunkt des Gelenks, hier des Oberschenkelknochens (Femur) bestimmt wurde. Hierzu wurden beispielsweise verschiedene Stellungen des Hüftknochens und der Hüftpfanne relativ zueinander bestimmt, indem z.B. Referenzsterne an dem registrierten Hüftknochen und der Hüftpfanne befestigt wurden und dann eine Bewegung des Hüftknochens relativ zur Hüftpfanne durchgeführt wurde. Vorzugsweise werden hier zusätzliche durch Fluoroskopiebilder erstellt, wie in EP 1 611 863 A beschrieben.

Vorzugsweise wird die präoperativ bestimmte Lage des Drehzentrums in das Datenverarbeitungsgerät eingelesen, so dass die präoperative Lage des Drehzentrums mit der postoperativen Lage des Drehzentrums, wie sie mittels des ICORS bestimmt wurde, verglichen werden kann.

Fig. 3 zeigt den Versatz OS, wie er sich durch eine medial-laterale Verschiebung des Drehzentrums ergibt. Weiter ist die Beinlängendifferenz LLD gezeigt, die sich durch eine kranial-kaudale Verschiebung des Drehzentrums ergibt. Man kann somit mit Hilfe des ICORS bestimmen, ob sich das Drehzentrum durch das Implantat, also durch die Operation, verändert hat oder gleich geblieben ist. Andere Implantate oder Implantatköpfe können dann verwendet werden, um eine bestmögliche Überstimmung zwischen dem präoperativen Drehzentrum und dem postoperativen Drehzentrum zu erzielen.

Die in Fig. 3 längs gezeichneten Linien sind die sog. mechanischen Achsen MA1 und MA2. Die mechanische Achse eines Oberschenkelknochens (Femur) ist durch den Mittelpunkt der Achse des Epicondylus des Femurs und durch das Drehzentrum definiert. Der Epicondylus ist der einem Gelenkknorren (Condylus) aufsitzende Knochenvorsprung für Muskelursprünge oder -ansätze. Am distalen Ende des Femurs befinden sich 2 Epicondylen, die mediale und die laterale Epicondyle. Die Verbindungslinie der beiden Epicondylen stellt die Epicondylenachse dar. Der Mittelpunkt der Verbindungslinie zwischen den beiden Epicondylen (laterale Condyle, mediale Condyle) definiert den distalen Endpunkt der mechanischen Femur-Achse.

Das erfindungsgemäße ICORS erlaubt somit die Bestimmung der mechanischen Achse MA1 nach der Operation und deren Vergleich mit der mechanischen Achse MA1 vor der Operation.

Ebenfalls erlaubt das erfindungsgemäße ICORS die Bestimmung der Vorwärtsneigung (Anteversion) des Femurs. Hierzu wird ein Winkel zwischen der Epicondylarachse und einer zweiten Achse bestimmt. Die zweite Achse ist durch das Drehzentrum und den Piriformis-Fossa-Punkt definiert. Der Piriformis-Fossa-Punkt ist durch die Registrierung des Oberschenkelknochens bekannt. Vorzugsweise sind die Daten, die den registrierten Knochen darstellen in der Datenverarbeitungseinrichtung 200 gespeichert. Vorzugsweise ist ein Referenzstern an dem Knochen angebracht, dessen relative Lage relativ zu dem Knochen bekannt ist, so dass aus der Detektion des Referenzsterns die Lage des Knochens und somit auch die Lage der Epicondylus-Achse und des Piriformis-Fossa-Punktes relativ zu dem ICORD bekannt ist.

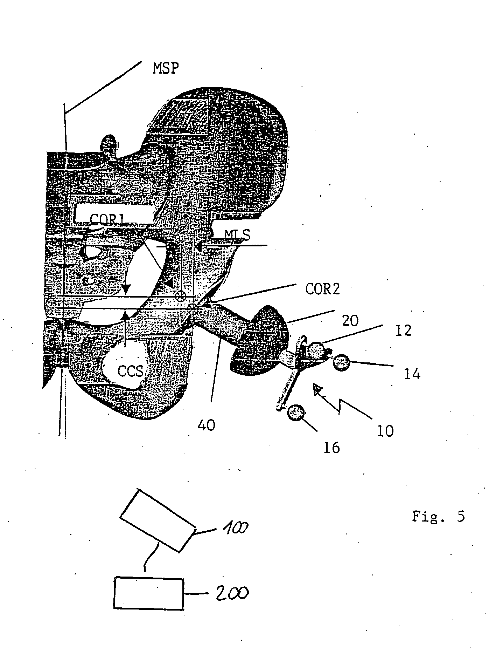

Fig. 4 zeigt eine zweite Ausführungsform eines erfindungsgemäßen ICORD. Bei dieser Ausführungsform ist ein Kontaktflächenglied 42 zum Anlegen an ein Implantat als konvexer Kopf ausgebildet. Dies ist also anders als bei der Ausführungsform gemäß Fig. 1, bei der die Anlagefläche konkav ausgebildet ist. Das ICORD gemäß Fig. 4 kann zum Bestimmen des Drehzentrums eines sphärisch konkaven Implantats verwendet werden. Wie in Fig. 4 gezeigt, verwendet das ICORD vorzugsweise Bauelemente des in Fig. 1 gezeigten ICORDS. Gleiche Teile sind wiederum mit gleichen Bezugszeichen versehen. Es ist somit wiederum die Markereinrichtung 10, der Träger 20 und die Anlagefläche 30 vorgesehen. Die Anlagefläche 30 dient hier als Anbringglied, das ortsfest und vorzugsweise lösbar mit dem Träger 20 verbindbar ist. In dem Anbringglied, d.h. in dessen konkave Vertiefung ist ein Einlegestück 40 eingebracht und damit vorzugsweise fest, aber insbesondere lösbar verbunden. Durch den modularen Aufbau des ICORD kann dieses sowohl zum Vermessen eines sphärisch konvexen Implantats als auch eines sphärisch konkaven Implantats verwendet werden. Das Einlegestück 40 erlaubt ein ICORD, das zum Vermessen eines sphärisch konvexen Implantats geeignet ist, umzubauen, und zwar in ein ICORD, das zum Vermessen eines sphärisch konkaven Implantats geeignet ist. Hierzu hat das Einlegestück 40 einen sphärisch konvexen Kopf 42, der in ein sphärisch konkaves Implantat eingeführt werden kann und an dieses satt angelegt werden kann. Durch die Verwendung von Einlegestücken unterschiedlicher Größe, insbesondere unterschiedlicher Kopfgestaltung (unterschiedlicher Kopfdurchmesser) ist es möglich, die verschiedensten Implantate mit unterschiedlichen sphärischen Radien (Krümmungsradien) zu bestimmen und insbesondere deren sphärischen Mittelpunkt zu bestimmen. Gleiches gilt im Übrigen für das ICORD gemäß Fig. 1, bei dem durch Verwenden unterschiedlicher Anlageflächenglieder mit unterschiedlichen sphärischen Radien der sphärischen Vertiefung die verschiedensten konvexen Implantate bestimmt, insbesondere deren Drehzentren bestimmt werden können.

Fig. 5 zeigt die Verwendung des ICORDS gemäß Fig. 4 bei der Bestimmung des sphärischen Mittelpunkts eines Implantats mit einer konkaven, sphärischen Vertiefung. In Fig. 5 wurde ein Hüftpfannenimplantat (nicht zu sehen) in die Hüfte implantiert. Das Hüftpfannenimplantat hat einen sphärischen Mittelpunkt COR2 und ist schalenförmig ausgebildet. Das CORD der Fig. 4 ist in das Hüftpfannenimplantat mit seinem Kopf 42 (nicht zu sehen) eingeführt, so dass das Einlegestück 40 aus der Hüfte hervorsteht. Auch bei dem in Fig. 5 gezeigten Fall lässt sich der Lageunterschied zwischen dem präoperativen Drehzentrum COR1 und dem (präoperativen) Drehzentrum COR2 nach der Operation bestimmen. Insbesondere lässt sich die kranial-kausale Verschiebung CCS und die medial-laterale Verschiebung MLS bestimmen. Die mittlere Sagitalebene des Hüftknochens ist mit MSP bezeichnet.

Das Implantatdrehzentrum-Bestimmungssystem ICORS der Fig. 5 umfasst ebenfalls eine Detektionseinrichtung 100, die als Kamera ausgebildet sein kann, und eine Datenverarbeitungseinrichtung 200.

Analog zur Fig. 3 gibt es auch hier wiederum zwei Alternativen zur Bestimmung des postoperativen Zentrums COR2. Gemäß einer Alternative ist die Geometrie des Kopfes 42 des Einlegestücks 40 bekannt, insbesondere ist die relative Lage des sphärischen Mittelpunkt des Kopfes 42 relativ zur Markereinrichtung 10 bekannt. "Bekannt" bedeutet in dieser Anmeldung, dass entsprechende Daten über die relative Lage in der Datenverarbeitungseinrichtung 200 vorliegen, insbesondere darin gespeichert sind, oder eingegeben wurden.

Gemäß einer alternativen Ausführungsform ist die relative Lage des sphärischen Mittelpunkts des Kopfes 42 relativ zur Markereinrichtung nicht bekannt. In diesem Fall werden dann vorzugsweise Schwenkbewegungen mit dem CORD durchgeführt, wobei auf eine satte Anlage des Kopfs 42 an der konkaven, sphärischen Oberfläche des Implantats geachtet werden muss. Die Schwenkbewegungen im Raum werden von der Detektionseinrichtung 100 detektiert. Sie erfolgen um den Drehpunkt COR2. Basierend auf der Annahme, dass sich die Markerkugel 12, 14 und 16 auf einer Kugeloberfläche bewegen, lässt sich somit der Mittelpunkt der Kugeloberfläche aus den detektierten Signalen bestimmen.

Ist das Drehzentrum COR2 bekannt, lässt er sich mit dem präoperativ bestimmten Drehzentrum COR1 vergleichen. Das präoperative Drehzentrum wurde beispielsweise genauso bestimmt, wie bei dem in Fig. 3 beschriebenen Fall, d.h. durch Bewegung des Oberschenkelknochens.

Ist das präoperative Drehzentrum COR1 bekannt, lässt sich durch Vergleich mit der bestimmten Lage des postoperativen Drehzentrums COR 2 sowohl die kranial-kaudale Verschiebung als auch die medial-laterale Verschiebung bestimmen.

## Patentansprüche

1. Implantatdrehzentrum-Bestimmungsgerät zum Bestimmen des Drehzentrums eines medizinischen Implantats
mit einem eine sphärische Oberfläche aufweisenden Anlageglied (20, 30) zum Anlegen eines sphärischen Implantats (50) und
mit Markern (12, 14, 16), die ortsfest relativ zum Anlageglied angeordnet sind.

2. Implantatdrehzentrum-Bestimmungsgerät nach Anspruch 1, bei welchem die sphärische Oberfläche des Anlageglieds (20, 30) konvex oder konkav ist.

3. Implantatdrehzentrum-Bestimmungsgerät nach Anspruch 1 oder 2, bei welchem das Anlageglied einen Träger (20) und ein davon lösbares Anlageflächenglied (30; 30, 40, 42) umfasst, wobei das Anlageflächenglied die sphärische Oberfläche aufweist.

4. Implantatdrehzentrum-Bestimmungsgerät nach Anspruch 3, bei welchem der Träger (20) mit einer Markereinrichtung (10) verbunden ist, die die Marker (12, 16, 16) aufweist.

5. Implantatdrehzentrum-Bestimmungsgerät, bei welchem das Anlageflächenglied eine Vertiefung mit konkaver sphärischer Fläche oder einen Fortsatz mit einem Kopf, der eine konvexe sphärische Oberfläche hat, aufweist.

6. Implantatdrehzentrum-Bestimmungsgerät zum Bestimmen des Drehzentrums eines medizinischen Implantats
mit einem Implantatdrehzentrum-Bestimmungsgerät nach einem der Ansprüche 1 bis 5,
mit einer Detektionseinrichtung (100) zum Detektieren der Lage der Marker und
mit einer Datenverarbeitungseinrichtung (200), die die Lage des Mittelpunkts der sphärischen Oberfläche des Anlageglieds (20, 30) basierend auf der detektierten Lage der Marker (12, 14, 16) und basierend auf
a) der bekannten relativen Lage des sphärischen Mittelpunkts der sphärischen Oberfläche des Anlageglieds (20, 30) relativ zu den Markern (12, 14, 16) bestimmt oder
b) den sphärischen Mittelpunkt der sphärischen Oberfläche des Anlageglieds basierend auf einer Vielzahl der detektierten Lagen der Marker, wobei die Vielzahl detektierter Lagen verschiedene relative Lagen des Implantatdrehzentrum-Bestimmungsgeräts relativ zum sphärischen Mittelpunkt der an dem Implantat anliegenden sphärischen Oberfläche des Anlageglieds (20, 30) darstellen.

7. Verfahren zum Bestimmen eines Implantatdrehzentrums mittels des Implantatdrehzentrum-Bestimmungsgeräts oder zum Planen einer derargien Bestimmung nach einem der Ansprüche 1 bis 5, wobei
a) ein Implantatdrehzentrum-Bestimmungsgerät ausgewählt wird, dessen sphärische Oberfläche einen Radius hat, der mit dem Radius der sphärischen Oberfläche des Implantats (50) übereinstimmt;
b) die sphärische Oberfläche des Implantatdrehzentrum-Bestimmungsgeräts an die komplementär ausgebildete sphärische Oberfläche des Implantats (50) angelegt wird;
c) mit der Detektionseinrichtung (100) mindestens eine Lage der Marker (12, 14, 16) detektiert wird; und
d) mittels der Datenverarbeitungseinrichtung (200) basierend auf einer Vielzahl von detektierten Lagen der Marker (12, 154, 16) oder basierend auf einer detektierten Lage der Marker (12, 14, 16) und basierend auf der bekannten relativen Lage zwischen sphärischem Mittelpunkt des Anlageglieds (20, 30) und den Markern (12, 14, 16) der sphärische Mittelpunkt des Implantats (50) bestimmt wird.
